(19) Europäisches Patentamt
European Patent Office
Office européen des brevets

(11) EP 2 368 542 B1

(12) EUROPEAN PATENT SPECIFICATION

(45) Date of publication and mention of the grant of the patent:
**21.03.2012 Bulletin 2012/12**

(51) Int Cl.:
***A61K 8/37*** *(2006.01)* ***A61K 8/46*** *(2006.01)*
***A61Q 9/04*** *(2006.01)* ***A61K 8/58*** *(2006.01)*
***A61K 8/92*** *(2006.01)*

(21) Application number: **10158068.6**

(22) Date of filing: **26.03.2010**

(54) **Depilatory method and kit**

Verfahren zur Depilation und Depilierausrüstung

Procédé d'épilation et kit d'épilation

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**28.09.2011 Bulletin 2011/39**

(60) Divisional application:
**11186193.6 / 2 409 679**

(73) Proprietor: **The Procter & Gamble Company**
**Cincinnati, OH 45202 (US)**

(72) Inventors:
- **Smith, Charles, Robert**
  **Henley on Thames, Oxfordshire RG9 3EB (GB)**
- **Hewlins, Stuart, Andrew**
  **Woking, Surrey GU24 9LW (GB)**
- **Goffe, Michael, John**
  **Egham, Surrey TW20 9LF (GB)**

(74) Representative: **Wilding, Richard Alan**
**Procter & Gamble**
**Technical Centres Ltd**
**Rusham Park**
**Whitehall Lane**
**Egham, Surrey TW20 9NW (GB)**

(56) References cited:
**EP-A1- 0 089 710** **WO-A1-99/02125**
**WO-A1-2004/096164** **WO-A2-2008/110745**
**US-A- 2 202 829** **US-A- 3 194 736**
**US-A- 4 546 112**

Remarks:
The file contains technical information submitted after the application was filed and not included in this specification

EP 2 368 542 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention relates to a depilatory method and kit.

BACKGROUND OF THE INVENTION

Depilatory compositions are cosmetic hair removal formulations. They comprise keratin reducing agents, which attack the disulphide bonds in hair to weaken it, such that subsequent gentle scraping and/or wiping completes severance of the hair from the skin and effects hair removal. Commercially, the most common keratin reducing agents are thioglycolates, which are typically formulated at high pH. An unwanted side effect of chemical depilation is that the depilatory composition comes into contact with and must have a relatively long residence time on skin to achieve effective hair removal and this long residence time combined with the alkaline conditions needed for effective hair removal may give rise to skin irritation.

The above problem has been recognized in the art. Reference is made to US 2004/0219118, which discloses treatment with a "lipophilic" material before application of a thioglycolate-based reactive depilatory compostion. Lipophilic materials exemplified in this patent application are oils, such as mineral oil. As shown hereinbelow, the present applicants have tested a range of lipophilic materials to determine their ability to prevent thioglycolate penetration and, thereby, their ability to reduce or prevent skin irritation Applicants have surprisingly found that oils, such as mineral oil, have no or a low ability to prevent thioglycolate penetration to the skin. There thus exists a nerd to develop a pre-treatment composition which better reduces skin irritation.

SUMMARY OF THE INVENTION

According to a first aspect of the invention, a method of removing hair from skin, preferably facial skin, is provided, comprising the steps of:

(a) applying a hydrophobic protective composition to an area of skin, preferably facial skin, on which unwanted hair is growing, the hydrophobic protective composition comprising from 1% to 60% of wax and from 40% to 99% oil by weight of the hydrophobic protective composition;
(b) applying a depilatory composition to the area of skin to which the hydrophobic protective composition has been applied, the depilatory composition comprising a keratin reducing agent.

According to a second aspect of the invention, a depilatory kit is provided, comprising:

(a) a hydrophobic protective composition, the hydrophobic protective composition comprising from 1% to 60% of wax and from 40% to 99% oil by weight of the hydrophobic protective composition;

(b) a depilatory composition comprising an effective amount of a keratin reducing agent.

BRIEF DESCRIPTION OF THE DRAWINGS

Fig. 1. is a schematic view of a Franz Cell apparatus.

DETAILED DESCRIPTION OF THE INVENTION

The hydrophobic protective composition used in the method and comprised within the kit according to the invention comprises from 1% to 60%, preferably from 2% to 24% and, more preferably, 3% to 15% wax by weight of the hydrophobic protective composition. As demonstrated by the Franz Cell data below, the presence of some wax in the hydrophobic protective composition surprisingly reduces penetration by thioglycolic acid in comparison with oils. Without wishing to be bound by theory, applicants believe that this may be because the wax militates against the tendency otherwise exhibited by oils to ball up on skin and therefore disrupt the barrier. The wax also ensures that a thin barrier of the hydrophobic protective composition can be evenly distributed across the skin, even at a low dosage per unit area. The wax may form a substantive barrier across the skin (enhanced by an amorphous mix of the oil & wax) that is chemically resistant to ingress from the thioglycolate (or other reducing) actives, therefore physically reducing the ability for the harsh chemistry to come into contact with the skin. This reduction in contact means that the stratum corneum may be maintained in a better state than if no barrier were present with correspondingly reduced signs of irritation, such

as erythema, tingling and stinging. A reduction of thioglycolic acid penetration of 45% or more according to the Franz Cell method may be shown to correlate to a significant and user-noticeable reduction in irritation.

At the same time as reducing contact between the depilatory active ingredient and the skin, the present compositions are observed not to noticeably reduce the ability of the depilatory composition to attack and degrade the unwanted hair growing on that skin. Why this should be is not understood, but it may simply be due to the fact that less of the hydrophobic protective composition adheres to the hairs than to the skin.

At wax levels above 60% by weight, the hydrophobic protective composition may become difficult to handle and apply and may also be brittle, crack and fall off the skin.

As used herein, the term "wax" includes, but is not limited to, any hydrophobic material that is:

- Solid at 25°C
- practically insoluble in water according to the United States' Pharmacopeia (USP) definition in 31/NF 26 Vol. 2 General Notices, Page Xvii. (which, according to that definition, means that more than 10,000 parts of water are needed to dissolve 1 part solute);
- has an onset temperature measured according to the DSC Method, defined hereinbelow, which is 10°C or greater; and
- comprises lipids, silicones or mixtures thereof.

The wax may comprise natural wax, synthetic wax, silicone wax, or mixtures thereof.

Non-limiting examples of suitable natural waxes include Abies Alba Leaf Wax, Acacia Dealbata Leaf Wax, Acacia Farnesiana Flower Wax, Beeswax, Ceresin, Cetyl Esters, Cistus Labdaniferus Flower Wax, Aurantium Amara (Bitter Orange) Flower Wax, Aurantium Dulcis (Orange) Peel Wax, Copernicia Cerifera (Camauba) Wax, Eclipta Prostrata Wax, Euphorbia Cerifera (Candelilla) Wax, Helichrysum Angustifolium Wax, Jasminum Officina le (Jasmine) Flower Wax. Jasminum Sambac (Jasmine) Flower Wax, Jojoba Esters, Jojoba Wax, Lanolin Wax, Lavandula Angustifolia (Lavender) Flower Wax, Lawsonia Inermis Wax, Mink Wax, Montan Acid Wax, Montan Wax, Myrica Cerifera (Bayberry) Fruit Wax, Ocimum Tenuiflorum Wax, Olive Wax, Oryza Sativa (Rice) Bran Wax, Ouricury Wax, Palm Kernel Wax, Persea Gratissima (Avocado) Wax, Pistacia Lentiscus Leaf Wax, Polianthes Tuberosa Flower Wax, Pyrus Malus (Apple) Peel Wax, Ribes Nigrum (Black Currant) Wax, Rosa Centifolia Flower Wax, Salvia Sclarea (Clary) Wax, Shellac Wax, Simmondsia Chinensis (Jojoba) Butter, Soft Olive Wax, Spent Grain Wax, Stipa Tenacissima Wax, Sunflower Seed Wax, Vegetable Wax, Vitis Vinifera (Grape) Leaf Wax and mixtures thereof.

Non-limiting examples of suitable synthetic waxes include Hydrogenated Japan Wax, Hydrogenated Jojoba Oil, Hydrogenated Jojoba Wax, Hydrogenated Microcrystalline Wax, Hydrogenated Rice Bran Wax, Hydrolyzed Beeswax, Microcrystalline Wax, Oxidized Beeswax, Oxidized Microcrystalline Wax, Ozokerite, Paraffin, PEG-6 Beeswax, PEG-8 Beeswax, PE G-12 Beeswax, PEG-20 Beeswax, PEG-12 Carnauba, Potassium Oxidized Microcrystalline Wax, Sulfurized Jojoba Oil, Synthetic Beeswax, Synthetic Candelilla Wax, Synthetic Carnauba, Synthetic Japan Wax, Synthetic Jojoba Oil, Synthetic Wax and mixtures thereof.

Non-limiting examples of suitable silicone waxes include DC2503 Cosmetic Wax, DC580 wax, DC AMS-C30 Cosmetic Wax, C30-45 Alkyl Methicone, DC Silkywax 10, Hexamethyldisiloxane, DC ST-Wax 30, C30-45 Alkyldimethylsilyl Polypropylsilsesquioxane, DC SW-8005 resin wax, C26 - 28 Alkyl Dimethicone, C26 - 28 Alkyl Methicone, Polyphenylsilsesquioxane and mixtures thereof.

Advantageously, the wax comprises beeswax, carnauba wax, candelilla wax, jojoba wax, paraffin wax, microcrystalline wax, ozokerite, arachidyl behenate, or mixtures thereof.

The hydrophobic protective composition used in the method and comprised within the kit according to the invention comprises one or more oils. As used herein, the term "oil" includes, but is not limited to any non-aqueous substance that is practically insoluble in water according to the United States' Pharmacopeia (USP) definition in 31/NF 26 Vol. 2 General Notices, Page Xvii. (which, according to that definition, means that more than 10,000 parts of water are needed to dissolve 1 part solute) and is liquid at 20°C.

The hydrophobic protective composition used in the method and comprised within the kit according to the invention comprises from 40-99%, preferably from 76% to 98% and, more preferably, from 85% to 97% oil by weight of the hydrophobic protective composition.

The oil may be selected from natural oil, synthetic oil, silicone oil and mixtures thereof.

Non-limiting examples of suitable natural oils include Acetylated Castor Oil, Acetylated Hydrogenated Castor Oil, Actinidia Chinensis (Kiwi), Seed Oil, Adansonia Digitata Oil, Aleurites Moluccana Seed Oil, Anacardium Occidentale (Cashew) Seed Oil, Arachis Hypogaea (Peanut) Oil, Arctium Lappa Seed Oil, Argania Spinosa Kernel Oil, Argemone Mexicana Oil, Avena Sativa (Oat) Kernel Oil, Bertholletia Excelsa Seed Oil, Borago Officinalis Seed Oil, Brassica Campestris (Rapeseed) Seed Oil, Calophyllum Tacamahaca Seed Oil, Camellia Japonica Seed Oil, Camellia Kissi Seed Oil, Camellia Oleifera Seed Oil, Canola Oil, Caprylic/Capric/Lauric Triglyceride, CapryliclCapric/Linoleic Triglyceride, Caprylic/Capric/Myristic/Stearic Triglyceride, Caprylic/Capric/Stearic Triglyceride, Caprylic/Capric Triglyceride, Carthamus Tinctorius (Hybrid Safflower) Seed Oil, Carthamus Tinctorius (Safflower) Seed Oil, Carum Carvi (Caraway) Seed Oil,

Carya Illinoensis (Pecan) Seed Oil, Castor Oil Benzoate, Chenopodium Quinoa Seed Oil, Cibotium Barometz Oil, Citrullus Vulgaris (Watermelon) Seed Oil, Cocos Nucifera (Coconut) Oil, Cod Liver Oil, Coffea Arabica (Coffee) Seed Oil, Coix Lacryma-Jobi (Job's Tears) Seed Oil, Corylus Americana (Hazel) Seed Oil, Corylus Avellana (Hazel) Seed Oil, Cucumis Sativus (Cucumber) Oil, Cucurbita Pepo (Pumpkin) Seed Oil, Daucus Carota Sativa (Carrot) Seed Oil, Elaeis Guineensis (Palm) Kernel Oil, Elaeis Guineensis (Palm) Oil, Gossypium (Cotton) Seed Oil, Helianthus Annuus (Hybrid Sunflower) Oil, Helianthus Annuus (Sunflower) Seed Oil, Hippophae Rhamnoides Oil, Human Placental Lipids, Hydrogenated Canola Oil, Hydrogenated Castor Oil, Hydrogenated Castor Oil Laurate, Hydrogenated Castor Oil Triisostearate, Hydrogenated Coconut Oil, Hydrogenated Cottonseed Oil, Hydrogenated C12-18 Triglycerides, Hydrogenated Fish Oil, Hydrogenated Lard, Hydrogenated Menhaden Oil, Hydrogenated Mink Oil, Hydrogenated Olive Oil, Hydrogenated Orange Roughy Oil, Hydrogenated Palm Kernel Oil, Hydrogenated Palm Oil, Hydrogenated Peanut Oil, Hydrogenated Rapeseed Oil, Hydrogenated Shark Liver Oil, Hydrogenated Soybean Oil, Hydrogenated Sunflower Seed Oil, Hydrogenated Tallow, Hydrogenated Vegetable Oil, Isatis Tinctoria Seed Oil, Juglans Regia (Walnut) Seed Oil, Lauric/Palmitic/Oleic Triglyceride, Umnanthes Alba (Meadowfoam) Seed Oil, Unum Usitatissimum (Linseed) Seed Oil, Lupinus Albus Seed Oil, Macadamia Integrifolia Seed Oil, Macadamia Ternifolia Seed Oil, Maleated Soybean Oil, Mangifera Indica (Mango) Seed Oil, Marmot Oil, Melaleuca Alternifolia (Tea Tree) Leaf Oil, Melia Azadirachta Seed Oil, Melissa Officina lis (Balm Mint) Seed Oil, Menhaden Oil, Mink Oil, Moringa pterygosperma Seed Oil, Mortierella Oil, Neatsfoot Oil, Nelumbium Speciosum Flower Oil, Nigella Sativa Seed Oil, Oenothera Biennis (Evening Primrose) Oil, Olea Europaea (Olive) Fruit Oil, Olea Europaea (Olive) Husk Oil, Orange Roughy Oil, Orbignya Cohune Seed Oil, Orbignya Oleifera Seed Oil, Oryza Sativa (Rice) Bran Oil, Oryza Sativa (Rice) Germ Oil, Ostrich Oil, Oxidized Corn Oil, Oxidized Hazel Seed Oil, Papaver Orientale (Poppy) Seed Oil, Passiflora Edulis Seed Oil, Persea Gratissima (Avocado) Oil, Pistacia Vera Seed Oil, Placental Lipids, Prunus Amygdalus Amara (Bitter Almond) Kernel Oil, Prunus Amygdalus Dulcis (Sweet Almond) Oil, Prunus Armeniaca (Apricot) Kernel Oil, Prunus Avium (Sweet Cherry) Seed Oil, Prunus Cerasus (Bitter Cherry) Seed Oil, Prunus Persica (Peach) Kernel Oil, Pyrus Malus (Apple) Oil, Ribes Nigrum (Black Currant) Seed Oil, Ricinus Communis (Castor) Seed Oil, Rosa Canina Fruit Oil, Rosa Moschata Seed Oil, Salmon Oil, Salvia Hispanica Seed Oil, Santalum Album (Sandalwood) Seed Oil, Sesamum Indicum (Sesame) Seed Oil, Shark Liver Oil, Solanum Lycopersicum (Tomato) Seed Oil, Soybean Lipid, Sphingolipids, Taraktogenos Kurzii Seed Oil, Telphairia Pedata Oil, Vegetable Oil, Vitis Vinifera (Grape) Seed Oil, Zea Mays (Corn) Germ Oil, Zea Mays (Corn) Oil and mixtures thereof.

Non-limiting examples of suitable synthetic oils include mineral oil, isopropyl pamitate, isopropyl stearate, isohexadecane, isododecane, polyglyceryl triisostearate and mixtures thereof. Non-limiting examples of suitable silicone oils include dimethicones (including partial esters of dimethicones and fatty acids derived from natural/synthetic oils), cyclomethicones, polydimethylsiloxanes (e.g. DC200 from Dow Coming), phenyl trimethicones, trimethyl pentaphenyl trisiloxane, dimethicone copolyols and mixtures thereof.

The hydrophobic protective composition used in the method and comprised within the kit according to the invention may comprise one or more triglycerides, the or each triglyceride having the following formula:

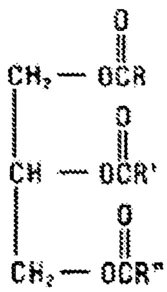

wherein R, R' and R" may be the same as or different from one or both of the others, wherein each of R, R' and R" is a fatty acid and wherein the or each triglyceride is solid at 25°C

Advantageously, the or each triglyceride has an onset temperature of less than 65°C as measured by Differential Scanning Calorimetry. At and above an onset temperature of 65°C, the composition may become increasingly difficult to apply and may, in addition, crack and fall off in use.

Suitable oils from which triglycerides may be formed from include, but are not limited to, the oils listed herein. Suitable fatty acids for formation of triglycerides include, but are not limited to, Myristoleic acid, Palmitoleic acid, Sapienic acid, Oleic acid, Linoleic acid, α-Linolenic acid ,Arachidonic acid , Eicosapentaenoic acid, Docosahexaenoic acid, Lauric acid ($C_{12}$), Myristic acid ($C_{14}$), Palmitic acid ($C_{16}$), Stearic acid ($C_{18}$), Arachidic acid ($C_{20}$) and mixtures thereof.

Specific sources of triglycerides suitable for inclusion in the hydrophobic protective composition include include

Butter, Shea Butter, Butyrospermum Parkii, Lipex Shea, Theobroma Cacao (Cocoa) Seed Butter, Cocoa Butter, Hydrogenated Shea Butter, Hydrogenated Cocoa Butter, Irvingia Gabonensis Kernel Butter, Tallow, Lard, Mangifera Indica (Mango) Seed Butter, Kokum Butter and mixtures thereof. The triglyceride(s) may fall under the definition of "wax" or "oil" as used herein and, in such a case, should be included as a wax or oil for the purposes of determining the proportions of wax or oil.

The hydrophobic protective composition used in the method and comprised within the kit according to the invention may comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof. These materials may fall under the definition of "wax" or "oil" as used herein and, in such a case, should be included as a wax or oil for the purposes of determining the proportions of wax or oil.

The hydrophobic protective composition used in the method and comprised within the kit according to the invention may include further ingredients such as, but not limited to metal oxides, organic and inorganic dyes, lakes, micas, flavourings, perfumes and mixtures thereof.

Any depilatory composition comprising a suitable keratin reducing agent may be used in the present method and included in the present kit. Non-limiting examples of suitable keratin reducing agents include: sulphide salts such as $Li_2S$, $Na_2S$, $K_2S$, MgS, CaS, SrS or BaS, hydrogen sulphide salts such as NaSH or KSH; thioglycol; thioglycerol; thioglycolamide; thioglycolhydrazide; thioglycolic acid; thioglycolate salts (such as potassium thioglycolate, calcium thioglycolate, ammonium thioglycolate, diammonium dithioglycolate, glyceryl monothioglycolate, or monoethanolamine thioglycolate); thiosalicylic acid; thiomalic acid; ammonium thiolactate; monoethanolamine thiolactate; dithioerythritol; 2-mercaptopropionic acid; 1,3-dithiopropanol; glutathione; dithiothreitol; cysteine; homocysteine; N-acetyl-L-cysteine and cysteamine. Advantageously, the keratin reducing agent is comprised within the depilatory composition in an amount from 0.3% to 20%, preferably from 0.8% to 15%, more preferably from 1% to 10% by weight of the depilatory composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, berylium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from about 0.01% to about 20%, preferably from about 0.1% to about 10% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted

for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or wanning agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a cream or lotion. Alternatively, it may be delivered on a substrate, such as a thin film of depilatory composition coated onto the substrate. The substrate may be configured in any suitable form, such as a strip, mask or patch.

In addition to the hydrophobic protective composition and the depilatory composition, the kit according to the second aspect of the invention may comprise one or more of:

(a) A make-up removal composition and/or a make-up removal wipe;

(b) Means for removal of the hydrophobic protective composition and the depilatory composition following use, which means may comprise one or more of a tool, such as a scraper or a spatula; or a wipe;

(c) A post-treatment composition skin care composition to be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

(d) Instructions regarding how to use the various elements of the kit, which instructions may comprise one or more elements of the method as defined herein.

Prior to applying the method or using the kit according to the present invention, a user should advantageously remove all make-up from the skin, to ensure good adherence and effective application of both the hydrophobic protective composition and the depilatory composition.

The method according to the first aspect of the invention comprises the step of applying the above-defined hydrophobic protective composition to an area of skin on which unwanted hair is growing. The area of skin may be located on any part of the human body, but is preferably on the face, more preferably on an area of skin adjacent to the vermillion lip and more preferably still on an area above the upper vermilion lip.

Advantageously, the hydrophobic protective composition is not just applied to the area to be depilated, but also to an immediately juxtaposing area thereabout (that is, the hydrophobic protective composition is applied to an area of skin which is greater than just the area which is to be depilated).

Advantageously, the user will apply from 0.3 - 2mg of hydrophobic protective composition per square centimetre of skin, preferably from 0.4 - 1mg/$cm^2$, more preferably from 0.4 to 0.7mg/$cm^2$.

Following application, the hydrophobic protective composition is advantageously massaged into the skin. Preferably, massaging is effected for at least 10 seconds, and, more preferably, massaging is effected as a circular motion. Without wishing to be bound by theory, it is believed that the hydrophobic protective composition may trap hair within it thereby shielding it from the to-be-applied depilatory composition; massaging may help to release the hairs from the skin and ensure improved access thereto by the depilatory composition.

The method according to the first aspect of the invention comprises the subsequent step of applying the above-defined depilatory composition to an area of skin on which unwanted hair is growing and to which hydrophobic protective composition has already been applied. Advantageously, the user will apply a layer of depilatory composition which is from 0.1mm to 5mm, preferably from 0.3 to 3mm, more preferably from 0.5 to 2mm in thickness.

Subsequently, according to the method of the first aspect of the invention, the depilatory composition is advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Subsequently, according to the method of the first aspect of the invention, the hydrophobic protective composition and the depilatory composition are advantageously removed. This may be achieved using one or more of a cotton wool ball, pad or wand, a tissue, a cloth, or a tool, such as a spatula or a scraper. Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

Differential Scanning Calorimetry (DSC) Melting Method

This method is the American Oil Chemists' Society Method Cj 1-94, as reapproved in 2009 and it determines the "onset temperature" (that is the temperature of onset of melting) of oils and fats by differential scanning calorimetry (DSC).

Apparatus

1. Aluminum capsules.

2. DSC instrument, capable of holding temperature at -60°C and achieving a temperature of 80°C.

Reagents

1. Indium, powder 60 mesh, 99.999%, such as Aldrich Chemical Co., Milwaukee, WI 53233, or equivalent.

2. n-Decane, 99+%, such as Aldrich Chemical Co., Milwaukee, WI 53233, or equivalent.

3. Methyl stearate, 99%, such as Aldrich Chemical Co., Milwaukee, WI 53233, or equivalent.

Procedure

1. Standardization of equipment—Proceed with the normal standardization using both indium and n-decane as reference standards. Follow instrument manual for adjustment to lock onto these two reference points and flatten the baseline slope as much as possible when empty pans are analyzed. Analyze the secondary standard (methyl stearate). Weigh 5 mg of the standard into the same kind of pan which will be used for the test portion (if hermetically sealed, it may be reused at a later date). Use the method sequence in Procedure, 2-7 to obtain the melting point onset (because of the high purity, only a 2 min hold is necessary for the standard after crystallization). Be certain that the heating rate during the definitive heating pattern is at 5°C/min. The melting point onset should be within ±2.00°C of 36.5°C. If not, recheck calibration.
*Note* be certain to use identical capsules for the test portion as those used for reference standards and the instrument blank reference.

2. Melt each test portion completely and weigh 7 ± 0.200 mg of each test portion into the same kind of capsule used for the blank and reference samples (aluminum) and seal to minimize oxidation and other changes.

3. Place capsules in DSC at room temperature.

4. Heat rapidly to 80°C and hold for 10 min.

5. Cool to -60°C at 10°C/min and hold for 30 min.

6. Heat to 80°C at 5°C/min.

7. Use the baseline obtained for an empty capsule analysis from the final melt segment of the program to define the position of the baseline under the sample peaks. Overlay the final melting curve of the test portion over the curve for the empty capsule with a flexible ruler or other curve guide to define the baseline of the test portion back to where it intersects the initial deviation of the melting curve from its baseline. The baseline beneath the test portion should be a continuation of the baseline where mere are no sample components present. If a shift has occurred in the heat capacity of the test portion after the melt, it will be evident relative to the baseline of the empty capsule. Have the instrument calculate the sigmoid baseline if it can, or connect the end of the peak point with the last point in which the test portion was in conjunction with the baseline of the empty capsule.

Results

Determine the onset temperature in °C, which, if not computer generated, is an extrapolation to baseline of the steepest slope of the principal peak.

Franz cell method

Principle and Scope:

This method is applicable for using Franz cell apparatus for the *in-vitro* assessment of penetration of thioglycolic acid (TGA) and its salts through a skin mimic after the application of a depilatory composition following pre-treatment with a hydrophobic protective composition.

Penetrated TGA is quantified using Reverse Phase High Performance (or Pressure) Liquid Chromatography (RP-HPLC) with external standard quantitation at 240nm.

Method

Reference is made to Figure 1 and to the reference numerals therein:

1. Prepare the Vitro-Skin (IMS Vitro-Skin®, Catalogue number: P&G1013, made by IMS Inc., Portland, Maine, USA) samples by cutting 8x6.2cm segments and placing them textured side up on the racks into a hydration chamber (manufactured & sold by IMS) containing a 14.7% glycerol solution. The hydration chamber should be sealed and the vitro-skin left to hydrate at room temperature and a humidity of 80.4% ±3.5% for 24 hours.

2. Prepare the receptor solution for the Franz-cell by mixing 1.90ml formic acid (98%wt+ Fluka, by Sigma Aldrich, or equivalent), 30ml acetonitrile (RP-HPLC grade) and 968.1ml water (RP-HPLC grade). Set up the static Franz cell (Permegear or equivalent, 15mm diameter unjacketed cell with a 12ml receptor volume) by clamping it in place over suitable stirrer plates (not shown) and add a small stirrer bar (6) to each cell, fill the receptor cell (2) to the brim with the required amount of receptor solution.

3. Once hydrated, remove a sheet of vitro-skin from the hydration chamber and lay textured side up on a clean flat surface then dose 100μl (~2mg/cm$^2$) of hydrophobic protective composition (not shown) onto the vitro-skin and spread evenly over the surface by rubbing for 30 seconds with a gloved finger.

4. Using a scalpel blade cut the vitro-skin segment (3) into two equal sections, each large enough to completely cover the top of the cell. Place the relevant size o-ring (5) (22mm, for the specified Franz-cell) onto each section of the vitro skin and dose to 150mg/cm$^2$ of depilatory composition (4) ("Veet Normal Skin Hair Removal Cream" or an equivalent (an equivalent being a composition comprising 3.7%wt thioglycolic acid)) into the centre then, using a glass rod, evenly spread the cream around the inside of the o-ring (5). Using tweezers pick up the vitro-skin segment and place the vitro-skin segment, depilatory and o-ring centrally over the receptor cell (2), place donor cell (1) over the top and clamp in place. Turn on stirrer plate and start 10 minute countdown timer. After 10 minutes; turn off stirrer and remove the clamp, donor cell (1) and vitro-skin segment and place the receptor solution in a suitable container for analysis.

5. A reference sample should also be run without hydrophobic protective composition treatment on the vitro-skin.

Remove a sheet of vitro-skin from the hydration chamber and lay textured side up on a clean flat surface. Repeat step 4 of the protocol to produce the reference sample.

Sample Analysis

For RP-HPLC analysis, prepare a 50mM Formic acid (98%+ Fluka) solution and mix 970ml of this solution with 30ml acetonitrile (HPLC grade) to act as a mobile phase during the analysis.

A reference standard solution should be made with a concentration of Calcium Thioglycolate Trihydrate of 0.94 mg/ml.

Install a Waters Atlantis T3 3μm 4.6 x 50mm column into the HPLC (although any silica- based $C_{18}$ reversed phase RP-HPLC column may be used), and ensure all solvent lines for the RP-HPLC are primed and free of leaks. Allow the mobile phase to circulate through the system for 25 minutes at 0.7mL/Min in order to equilibrate the column. Detection of the thioglycolic acid is via UV spectroscopy.

The RP-HPLC conditions are as follows:

- Injection volume: 20 μL
- Mobile phase flow rate: 0.70 ml/min
- Run time: 10 minutes
- UV Detection wavelength: 240 nm
- Column temperature: 35°C
- UV sampling rate: ≥ 5 per second
- Retention time: Thioglycolic Acid - 2.5 min

Calculations:

Calculate the concentration of Thioglycolic Acid in the sample

$$\text{concentration (mg/ml)} = \frac{\textbf{weight of std (mg) x purity}}{\textbf{25}} \times \frac{\textbf{3}}{\textbf{25}}$$

Calculate the concentration of thioglycolic acid in the sample using the following formula:

$$\text{concentration (mg/ml)} = \mathbf{A/B \times C \times E/F}$$

Where,
A = Peak Area of Thioglycolic Acid Sample
B = Average Peak Area of Thioglycolic Acid Standard
C = Thioglycolic Acid final STD concentration in mg/ml (0.94 mg/ml)
E = Molecular weight of Thioglycolic acid (92.12g/mol)
F = Molecular weight of Calcium thioglycolate (184.23g/mol)

The efficacy of the barrier (resistance to TGA penetration) can be calculated as a percentage decrease in TGA in the receptor solution:

$$\%\text{reduction} = \frac{\text{concentration without barrier} - \text{concentration with barrier}}{\text{concentration without barrier}} \times 100$$

For example, if TGA in solution without hydrophobic protective composition = 75μg/ml and TGA in solution with barrier = 15 μg/ml

$$\%reduction = \frac{75 - 60}{75} \times 100 = \mathbf{85\%}$$

A reduction of TGA penetration of 45% or more is believed to correlate to a significant and user-noticeable reduction in irritation.

Examples

The following compositions were made by heating all elements of the composition to the melting temperature of the wax and then mixing until a homogenous mixture was obtained. The compositions were then tested using the Franz Cell method defined above.

| Example | Composition | %Reduction in Thioglycolic Acid Penetration According to the Franz Cell Method |
|---|---|---|
| Inventive Example 1 | 6% beeswax<br>6% ozokerite<br>88% mineral oil | 99.0 |
| Inventive Example 2 | 12% beeswax<br>88% DC245 (Dow Coming) | 95.0 |
| Inventive Example 3 | 6% beeswax<br>94% mineral oil | 95.3 |
| Inventive Example 4 | 6% beeswax<br>94% coconut oil | 85.9 |
| Inventive Example 5 | 12% beeswax<br>88% DC200 (Dow Coming, 50cSt) | 94.1 |
| Comparative Example 1 | 100% mineral oil | 25.0 |
| Comparative Example 2 | 100% Sunflower Seed oil | 10.8 |
| Comparative Example 3 | 100% olive oil | 0.0 |
| Comparative Example 4 | 12% shea butter<br>88% mineral oil | 20.1 |
| Comparative Example 5 | 12% cocoa butter<br>88% mineral oil | 21.1 |

As demonstrated by the Franz Cell data, oils, on their own (see Comparative Examples 1 and 2), or mixed with small amounts of triglycerides (see Comparative Examples 3 and 4), such as shea or cocoa butter, provide little to no barrier to thioglycolic acid, whereas the addition of a small amount of wax surprisingly increases the barrier to penetration by a significant amount (see Inventive Examples 1-5).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### Claims

**1.** A method of removing hair from skin, preferably facial skin, comprising the steps of:

(a) applying a hydrophobic protective composition to an area of skin, preferably facial skin, on which unwanted hair is growing, the hydrophobic protective composition comprising from 1% to 60% of wax and from 40% to

99% oil by weight of the hydrophobic protective composition.
(b) applying a depilatory composition to the area of skin to which the hydrophobic protective composition has been applied, the depilatory composition comprising a keratin reducing agent.

2. The method of claim 1, wherein the amount of hydrophobic protective composition applied to the skin is from 0.3 - 2mg/cm$^2$, preferably from 0.4 - 1mg/cm$^2$, more preferably from 0.4 to 0.7mg/cm$^2$.

3. The method of claim 1 or 2, wherein the depilatory composition is applied as a layer to the skin which has been pre-treated with hydrophobic protective composition, wherein the layer has a thickness from 0.1mm to 5mm, preferably from 0.3 to 3mm, more preferably from 0.5 to 2mm.

4. The method of any of claims 1 to 3, comprising the following additional step between step (a) and step (b):

(a1) massaging the hydrophobic protective composition into the skin for at least 10 seconds.

5. The method of any preceding claim, comprising the following additional step immediately following step (b):

(c) leaving the depilatory composition in place on the hydrophobic protective composition for a period of at least 1 minute, preferably from 1 to 10 minutes, more preferably from 3 to 10 minutes.

6. The method of claim 5, comprising the following additional step immediately following step (c):

(d) removing both the hydrophobic protective composition and the depilatory composition from the skin, optionally scraping, wiping or rubbing it off.

7. The method of any preceding claim, wherein the hydrophobic protective composition comprises from 2% to 24%, preferably from 3% to 15% wax by weight of the hydrophobic protective composition.

8. The method of any preceding claim, wherein the wax comprises natural wax, synthetic wax, silicone wax, or mixtures thereof.

9. The method of any preceding claim, wherein the keratin reducing agent comprises a thioglycolate salt, preferably potassium or calcium thioglycolate, or mixtures thereof.

10. A depilatory kit comprising:

(a) a hydrophobic protective composition, the hydrophobic protective composition comprising from 1% to 60% of wax and from 40% to 99% oil by weight of the hydrophobic protective composition.
(b) a depilatory composition comprising an effective amount of a keratin reducing agent.

11. The depilatory kit of claim 10, wherein the depilatory composition is in the form of a cream or lotion.

12. The depilatory kit of claim 10, wherein the depilatory composition is disposed on a substrate.

13. The depilatory kit of any of claims 10 to 12, additionally comprising a tool, such as a scraper or a spatula, or a wipe.

**Patentansprüche**

1. Verfahren zum Entfernen von Haar von Haut, bevorzugt Gesichtshaut, das die folgenden Schritte umfasst:

(a) Auftragen einer hydrophoben Schutzzusammensetzung auf einen Bereich der Haut, vorzugsweise der Gesichtshaut, auf dem unerwünschtes Haar wächst, wobei die hydrophobe Schutzzusammensetzung von 1 Gew.-% bis 60 Gew.-% Wachs und von 40 Gew.-% bis 99 Gew.-% der hydrophoben Schutzzusammensetzung Öl umfasst;
(b) Auftragen einer Enthaarungszusammensetzung auf den Bereich von Haut, auf den die hydrophobe Schutzzusammensetzung aufgetragen wurde, wobei die Enthaarungszusammensetzung ein Keratinreduktionsmittel umfasst.

**2.** Verfahren nach Anspruch 1, wobei die Menge an hydrophober Schutzzusammensetzung, die auf die Haut aufgetragen wird, von 0,3 - 2 mg/cm$^2$, bevorzugt von 0,4 - 1 mg/cm$^2$, mehr bevorzugt von 0,4 bis 0,7 mg/cm$^2$ beträgt.

**3.** Verfahren nach Anspruch 1 oder 2, wobei die Enthaarungszusammensetzung als eine Schicht auf die Haut, die mit der hydrophoben Schutzzusammensetzung vorbehandelt wurde, aufgetragen wird, wobei die Schicht eine Dicke von 0,1 mm bis 5 mm, bevorzugt von 0,3 bis 3 mm, mehr bevorzugt von 0,5 bis 2 mm aufweist.

**4.** Verfahren nach einem der Ansprüche 1 bis 3, das zwischen Schritt (a) und Schritt (b) den folgenden zusätzlichen Schritt umfasst:

(a1) Einmassieren der hydrophoben Schutzzusammensetzung in die Haut für mindestens 10 Sekunden.

**5.** Verfahren nach einem der vorstehenden Ansprüche, das unmittelbar nach Schritt (b) den folgenden zusätzlichen Schritt umfasst:

(c) Belassen der Enthaarungszusammensetzung auf der hydrophoben Schutzzusammensetzung für einen Zeitraum von mindestens 1 Minute, bevorzugt von 1 bis 10 Minuten, mehr bevorzugt von 3 bis 10 Minuten.

**6.** Verfahren nach Anspruch 5, das unmittelbar nach Schritt (c) den folgenden zusätzlichen Schritt umfasst:

(d) Entfernen sowohl der hydrophoben Schutzzusammensetzung als auch der Enthaarungszusammensetzung von der Haut, wahlweise durch Abschaben, Abwischen oder Abreiben.

**7.** Verfahren nach einem der vorstehenden Ansprüche, wobei die hydrophobe Schutzzusammensetzung von 2 Gew.-% bis 24 Gew.-%, bevorzugt von 3 Gew.-% bis 15 Gew.-% der hydrophoben Schutzzusammensetzung Wachs umfasst.

**8.** Verfahren nach einem der vorstehenden Ansprüche, wobei der Wachs natürliches Wachs, synthetisches Wachs, Silikonwachs oder Mischungen davon umfasst.

**9.** Verfahren nach einem der vorstehenden Ansprüche, wobei das Keratinreduktionsmittel ein Thioglycolatsalz, vorzugsweise Kalium- oder Kalziumthioglycolat oder Mischungen davon umfasst.

**10.** Enthaarungsset, umfassend:

(a) eine hydrophobe Schutzzusammensetzung, die von 1 Gew.-% bis 60 Gew.-% Wachs und von 40 Gew.-% bis 99 Gew.-% der hydrophoben Schutzzusammensetzung Öl umfasst;
(b) eine Enthaarungszusammensetzung, die eine wirksame Menge eines Keratinreduktionsmittels umfasst.

**11.** Enthaarungsset nach Anspruch 10, wobei die Enthaarungszusammensetzung in Form einer Creme oder Lotion vorliegt.

**12.** Enthaarungsset nach Anspruch 10, wobei die Enthaarungszusammensetzung auf einem Substrat angeordnet ist.

**13.** Enthaarungsset nach einem der Ansprüche 10 bis 12, außerdem umfassend ein Werkzeug wie einen Schaber oder einen Spachtel oder ein Wischtuch.

## Revendications

**1.** Procédé d'élimination des poils de la peau, de préférence la peau du visage, comprenant les étapes consistant à :

(a) appliquer une composition protectrice hydrophobe sur une zone de peau, de préférence la peau du visage, sur laquelle des poils indésirables poussent, la composition protectrice hydrophobe comprenant de 1 % à 60 % de cire et de 40 % à 99 % d'huile en poids de la composition protectrice hydrophobe ;
(b) appliquer une composition dépilatoire sur la zone de peau sur laquelle la composition protectrice hydrophobe a été appliquée, la composition dépilatoire comprenant un agent réducteur de kératine.

**2.** Procédé selon la revendication 1, dans lequel la quantité de composition protectrice hydrophobe appliquée sur la peau va de 0,3 à 2 $mg/cm^2$, de préférence de 0,4 à 1 $mg/cm^2$, plus préférablement de 0,4 à 0,7 $mg/cm^2$.

**3.** Procédé selon la revendication 1 ou 2, dans lequel la composition dépilatoire est appliquée en tant que couche sur la peau qui a été prétraitée avec la composition protectrice hydrophobe, dans lequel la couche a une épaisseur allant de 0,1 mm à 5 mm, de préférence de 0,3 à 3 mm, plus préférablement de 0,5 à 2 mm.

**4.** Procédé selon l'une quelconque des revendications 1 à 3, comprenant l'étape supplémentaire suivante entre l'étape (a) et l'étape (b) :

(a1) faire pénétrer la composition protectrice hydrophobe dans la peau en massant pendant au moins 10 secondes.

**5.** Procédé selon l'une quelconque des revendications précédentes, comprenant l'étape supplémentaire suivante immédiatement après l'étape (b) :

(c) laisser la composition dépilatoire en place sur la composition protectrice hydrophobe pendant une période d'au moins 1 minute, de préférence de 1 à 10 minutes, plus préférablement de 3 à 10 minutes.

**6.** Procédé selon la revendication 5, comprenant l'étape supplémentaire suivante immédiatement après l'étape (c) :

(d) éliminer à la fois la composition protectrice hydrophobe et la composition dépilatoire de la peau, facultativement en la raclant, l'essuyant ou la frottant.

**7.** Procédé selon l'une quelconque des revendications précédentes, dans lequel la composition protectrice hydrophobe comprend de 2 % à 24 %, de préférence de 3 % à 15 % de cire en poids de la composition protectrice hydrophobe.

**8.** Procédé selon l'une quelconque des revendications précédentes, dans lequel la cire comprend une cire naturelle, une cire synthétique, une cire de silicone, ou leurs mélanges.

**9.** Procédé selon l'une quelconque des revendications précédentes, dans lequel l'agent réducteur de kératine comprend un sel de thioglycolate, de préférence du thioglycolate de potassium ou calcium, ou leurs mélanges.

**10.** Trousse dépilatoire comprenant:

(a) une composition protectrice hydrophobe, la composition protectrice hydrophobe comprenant de 1 % à 60 % de cire et de 40 % à 99 % d'huile en poids de la composition protectrice hydrophobe ;
(b) une composition dépilatoire comprenant une quantité efficace d'un agent réducteur de kératine.

**11.** Trousse dépilatoire selon la revendication 10, dans laquelle la composition dépilatoire est sous la forme d'une crème ou d'une lotion.

**12.** Trousse dépilatoire selon la revendication 10, dans laquelle la composition dépilatoire est disposée sur un substrat.

**13.** Trousse dépilatoire selon l'une quelconque des revendications 10 à 12, comprenant en outre un outil, tel qu'un racloir ou une spatule, ou une lingette.

Fig.1.

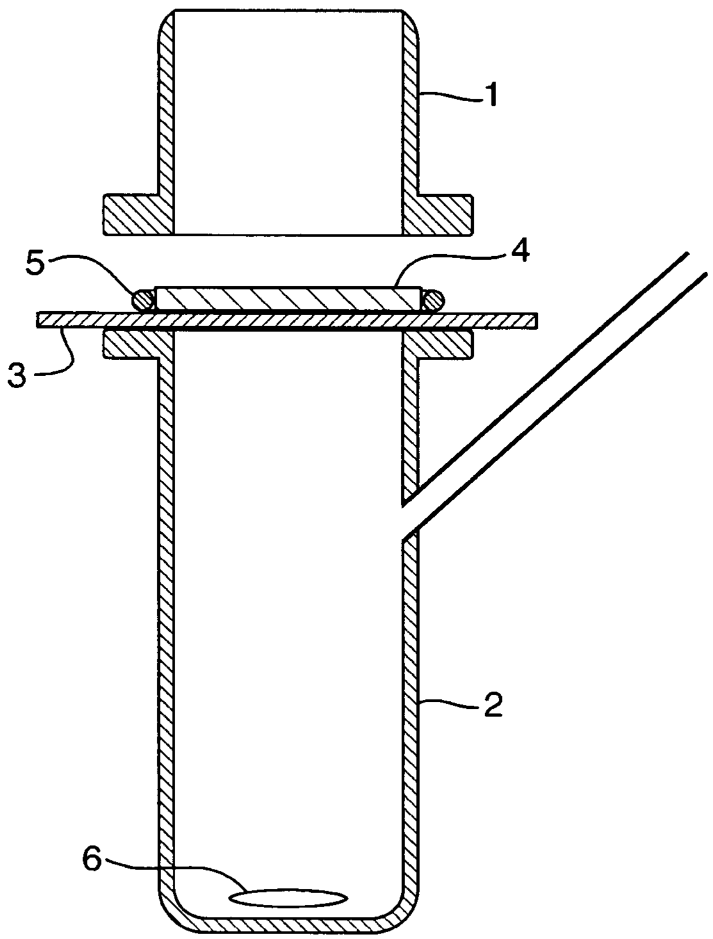

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040219118 A

### Non-patent literature cited in the description

- The Encyclopaedia of Polymers and Thickeners for Cosmetics. University of Southern Mississippi